# EUROPEAN PATENT SPECIFICATION

(11) **EP 1 290 182 B1**
(45) Date of publication and mention of the grant of the patent: **22.12.2010**
(21) Application number: 01929118.6
(22) Date of filing: 11.05.2001
(51) Int. Cl.: A61K 31/133, A61P 35/00

(54) **SPHINGOSINE KINASE AND USES THEREOF**
SPHINGOSINKINASE UND DEREN VERWENDUNGEN
SPHINGOSINE KINASE ET SES UTILISATIONS

(30) Priority: 11.05.2000 AU PQ744700
(43) Date of publication of application: 12.03.2003
(73) Proprietor: MEDVET SCIENCE PTY. LTD., Thebarton, S.A. 5031 (AU)
(72) Inventor: VADAS, Mathew, Stirling, S.A. 5152 (AU); GAMBLE, Jennifer, Stirling, S.A. 5152 (AU); XIA, Pu, Magill, S.A. 5072 (AU); WANG, Lijun, Magill, S.A. 5072 (AU)
(74) Representative: Leathley, Anna Elisabeth
(86) International application number: PCT/AU2001/000539
(87) International publication number: WO 2001/085953

(56) References cited:
- WO-A-00/00207
- WO-A-00/70028
- WO-A-01/31029
- WO-A-01/74837
- WO-A-99/12533
- WO-A-99/61581
- DATABASE WPI Section Ch, Week 199837 Derwent Publications Ltd., London, GB; Class B02, AN 1998-433849 XP002353566 & JP 10 182627 A (SANKYO CO LTD) 7 July 1998 (1998-07-07)
- XIA P ET AL.: "An oncogenic role of shingosine kinase" CURRENT BIOLOGY, vol. 10, no. 23, 17 November 2000 (2000-11-17), pages 1527-1530, XP002350767
- XIA ET AL.: 'High density lipoproteins (HDL) interrupt the sphingosine kinase signaling pathway' JOURNAL OF BIOLOGICAL CHEMISTRY vol. 274, 1999, pages 33143 - 33147, XP002962413
- NIWA ET AL.: 'Tumor necrosis factor-alpha-mediated signal transduction in human neutrophils: involvement of sphingomyelin metabolites in the priming effect of TNF-alpha on the FMLP-stimulated superoxide production' LIFE SCIENCES vol. 66, March 2000, pages 245 - 256, XP002962410
- HAUSER ET AL.: 'Role of ceramide in mitogenesis induced by exogenous sphingoid bases' JOURNAL OF BIOLOGICAL CHEMISTRY vol. 269, 1994, pages 6803 - 6809, XP002962412
- NAKAMURA ET AL.: 'Survival by Mac-1 mediated adherence and anoikis in phorbol ester-treated HL-60 cells' JOURNAL OF BIOLOGICAL CHEMISTRY vol. 273, 1998, pages 15345 - 15351, XP002962409
- XIA ET AL.: 'Tumor necrosis factor-alpha induces adhesion molecule expression through the sphingosine kinase pathway' PROC. NATL. ACAD. SCI. USA vol. 95, 1998, pages 14196 - 14201, XP002962411
- XIA ET AL.: 'Activation of sphingosine kinase by tumor necrosis factor-alpha inhibits apoptosis in human endothelial cells' JOURNAL OF BIOLOGICAL CHEMISTRY vol. 274, 1999, pages 34499 - 34505, XP002962408
- PYNE ET AL.: 'Sphingosine 1-phosphate signalling in mammalian cells' BIOCHEM. J. vol. 349, September 2000, pages 385 - 402, XP000939036

## Description

### FIELD OF THE INVENTION

The present invention relates generally to a method of modulating the growth of cells and, more particularly, to a method of down-regulating the growth of neoplastic cells wherein the neoplastic cells have become transformed by upregulation of the oncogene Ras. The present invention is useful, *inter alia,* in the therapeutic and/or prophylactic treatment of cancers such as solid cancers such as cancers of the colon, stomach, lung, brain, bone, oesophagus, pancreas, breast, ovary or uterus.

### BACKGROUND OF THE INVENTION

Bibliographic details of the publications referred to by author in this specification are collected at the end of the description.

The reference to any prior art in this specification is not, and should not be taken as, an acknowledgment or any form of suggestion that that prior art forms part of the common general knowledge in Australia.

Sphingosine kinase is a key regulatory enzyme in a variety of cellular responses. Sphingosine-1-phosphate is known to be an important second messenger in signal transduction (Meyer *et al.,* 1997). It is mitogenic in various cell types (Alessenko, 1998) and appears to trigger a diverse range of important regulatory pathways including prevention of ceramide-induced apoptosis (Culliver *et al.,* 1996), mobilisation of intracellular calcium by an IP₃-independant pathway, stimulation of DNA synthesis, activation of mitogen-activated protein (MAP) kinase pathway, activation of phospholipase D, and regulation of cell motility (for reviews see (Meyer *et al.,* 1997; Spiegal *et al.,* 1998; Igarashi, 1997)).

Recent studies (Xia *et al.,* 1998) have shown that sphingosine-1-phosphate is an obligatory signalling intermediate in the inflammatory response of vascular endothelial cells to to tumour necrosis factor-α(TNFα). In spite of its obvious importance, very little is known of the mechanisms that control cellular sphingosine-1-phosphate levels. It is known that sphingosine-1-phosphate levels in the cell are mediated largely by its formation from sphingosine by sphingosine kinase, and to a lesser extent by its degradation by endoplasmic reticulum-associated sphingosine-1-phosphate lyase and sphingosine-1-phosphate phosphatase (Spiegal *et al.,* 1998). Basal levels of sphingosine-1-phosphate in the cell are generally low, but can increase rapidly and transiently when cells are exposed to mitogenic agents. This response appears correlated with an increase in sphingosine kinase activity in the cytosol and can be prevented by addition of the sphingosine kinase inhibitory molecules *N,N-*dimethylsphingosine and DL-threo-dihydrosphingosine. This indicates that sphingosine kinase is an important molecule responsible for regulating cellular sphingosine-1-phosphate levels. This places sphingosine kinase in a central and obligatory role in mediating the effects attributed to sphingosine-1-phosphate in the cell.

Sphingosine kinase is speculated to play a role in a number of cellular activities including inflammation, calcium mobilisation, cell motility and adhesion molecule expression. However, the precise nature of the cellular activities which are so regulated and the role and mechanistic actions of sphingosine kinase in this regard are only just beginning to be understood. Many of the signals leading to modulation of various cellular activities have not been precisely defined. Elucidating these cellular signalling mechanisms is necessary for the development of therapeutic and prophylactic strategies to disease conditions involving aberrant or otherwise unwanted cellular activities.

JP182627 describes two sphingosine kinase inhibitors. WO99/61581 discloses nucleic acids encoding sphingosine kinase (SPHK) and methods for use in producing sphingosine-1-phosphate (SPP), screening for inhibitors of SPHK, and as a therapeutic agent. WO9912533 discloses a method of modulating endothelial cell activity and endothelial cell adhesion molecule expression and agents useful for the same. The method is applicable to treating coronary heart disease by preventing or reducing endothelial cell adhesion molecule expression, and modulation of sphingosine kinase is discussed in this document. WO00/00207 discloses a method of treating a hyperproliferative disorder by administering in combination, a treatment effective amount of: (a) a ceramide-generating retinoid such as fenretinide or a pharmaceutically acceptable salt thereof; and (b) at least one (and in certain embodiments at least two) ceramide degredation inhibitor, such as compounds selected from the group consisting of (i) glucosylceramide synthesis inhibitors, (ii) sphingosine-1-phosphate synthesis inhibitors, and (iii) protein kinase C inhibitors.

In work leading up to the present invention, the inventors have determined that the signalling cascade stimulated by the lipid kinase, sphingosine kinase, plays a major role in oncogenesis and is, in fact, by itself oncogenic when its level of activity is too high. Even in light of preliminary data indicating a role for sphingosine kinase in various cellular activities, the oncogenic activity of sphingosine kinase is a surprising and completely unexpected function attributable to this molecule. Identification of the link between sphingosine kinase and tumour pathogenesis permits the rational design of therapeuticand/or prophylactic regimes for modulating cell growth and, further, the identification of a range of molecules for use in the modulation of cell growth.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

Disclosed herein is a method of down-regulating the proliferation of a neoplastic cell, which has become transformed by upregulation of the oncogene Ras said method comprising contacting said neoplastic cell with an effective amount of an agent for a time and under conditions sufficient to inhibit sphingosine kinase.

Thus, one aspect of the invention provides an *in vitro* method of downregulating the proliferation of a neoplastic cell, wherein said neoplastic cell has become transformed by upregulation of the oncogene Ras, said method comprising contacting said cell with an effective amount of an agent for a time and under conditions sufficient to inhibit human sphingosine kinase.

Further disclosed is the use of an agent capable of modulating the functional activity of sphingosine kinase in the manufacture of a medicament for the modulation of cell growth in a mammal wherein down-regulation of the functional activity of said sphingosine kinase down-regulates the subject cell growth.

Thus a further aspect of the invention provides an agent capable of inhibiting human sphingosine kinase for use in the treatment of a neoplastic condition in a human wherein said neoplasia has become transformed by upregulation of the oncogene Ras.

Pharmaceutical compositions comprising a modulatory agent as hereinbefore defined and one or more pharmaceutically acceptable carriers and/or diluents may be used in methods or uses of the invention. Said modulatory agents are referred to as the active ingredients.

Diagnostic methodology based on screening individuals for the presence of sphingosine kinase or mRNA or protein or the specific forms of sphingosine kinase which are transcribed and/or translated by a given population of cells is also described. The screening methodology may be directed to qualitative and/or quantitative sphingosine kinase analysis. This is particularly useful, for example, for determining whether a given individual is predisposed to or resistant to diseases/disorders in which aberrant, unwanted or otherwise inappropriate cell growth is a component of the disease state and/or is predisposed or resistant to the development of certain forms of aberrant, unwanted or otherwise inappropriate cell growth.

### BRIEF DESCRIPTION OF THE DRAWINGS

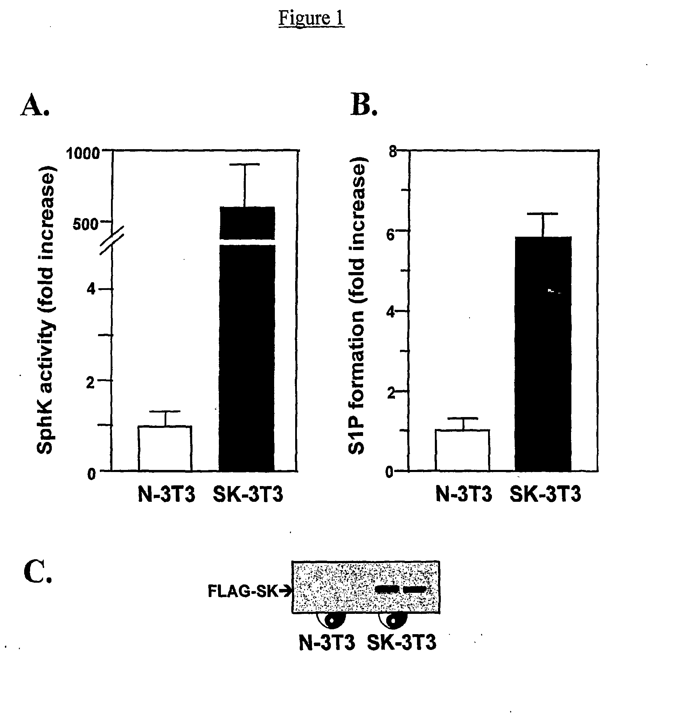

**Figure 1** is a graphical representation of the overexpression of SphK in NIH 3 T3 cells. **(A)** Cytosolic SphK activity was measured in the stable transfected cells with pcDNA3-SphK-FLAG (SK-3T3) or empty vector alone (N-3T3). **(B)** Intracellular S1P levels were measured in [³H]sphingosine-labelled SK-3T3 or N-3T3 cell pools. **(C)** Proteins from soluble cell lysates were probed with anti-FLAG monoclonal antibodies (M2, Kodak).

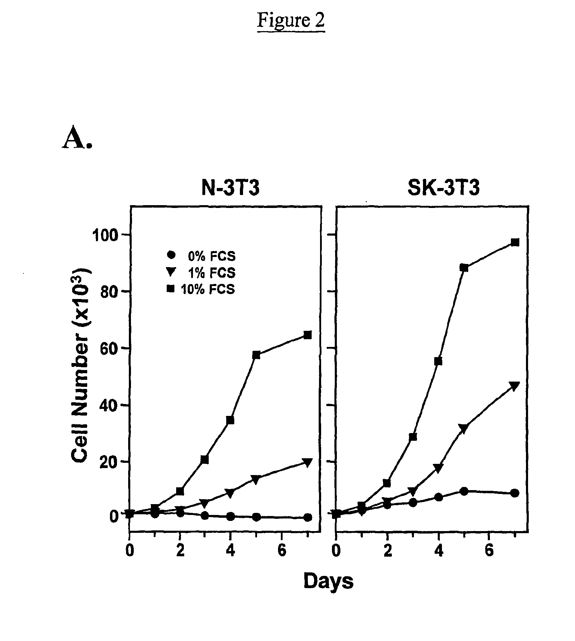

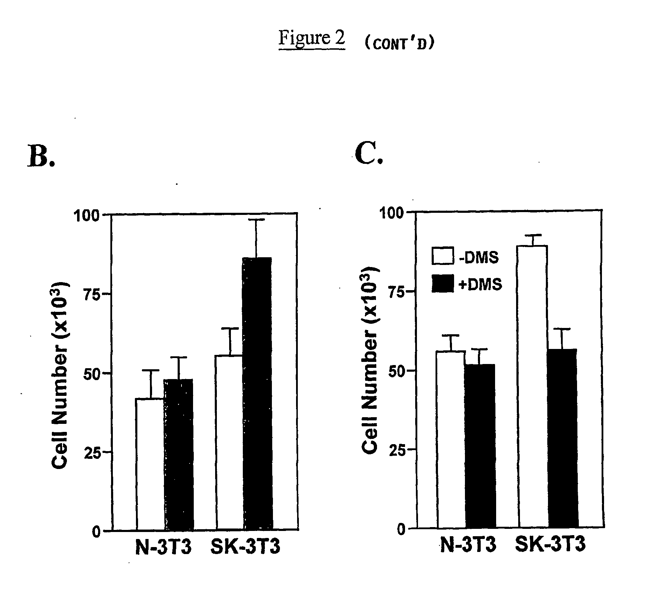

**Figure 2** is a graphical representation of SphK overexpression accelerates proliferation in NIH 3T3 cells. **(A)** Growth curves reveal that SK-3T3 cells (right panel) proliferate more rapid than N-3T3 cells (left panel). Values are the mean of triplicate determinations and similar results were obtained in three independent experiments. **(B)** Cell growth at saturation density. Cells were counted at absolute confluence (open bars) and 24 h later of confluence (grey bars), respectively. **(C)** DMS inhibits SphK overexpression induced proliferation. Equal numbers of N-3T3 and SK-3T3 cells were cultured in DMEM containing 10% serum in the presence or absence of DMS (2.5 µM) for 5 days. Media was replaced every day. Data in **(B)** and **(C)** are the means ± S.D. from three independent experiments done in triplicate.

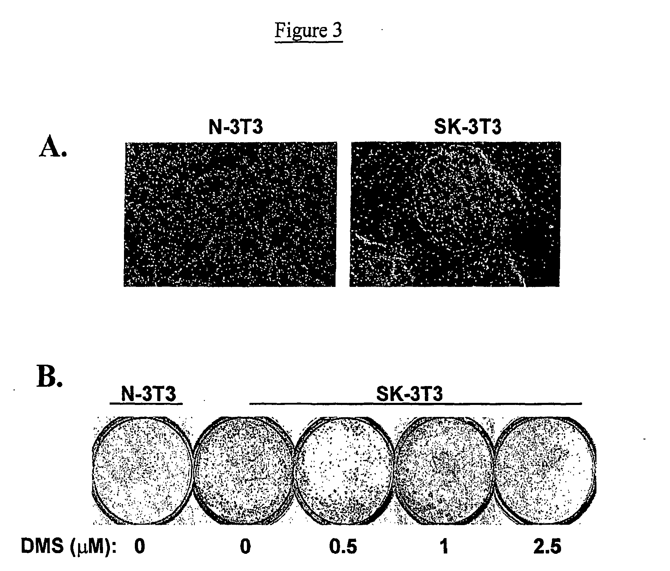

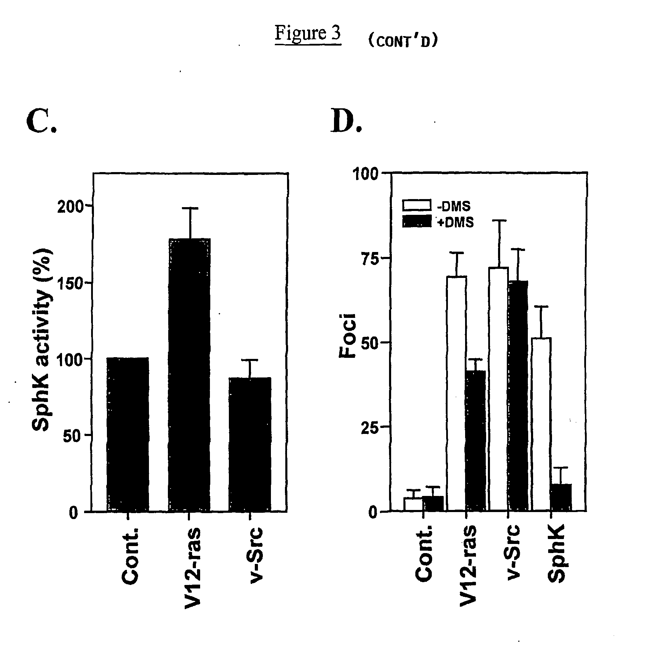

**Figure 3** is an image of overexpression of SphK induced NIH 3T3 cell transformation. **(A)** Focus formation of NIH 3T3 cell transfecants. Cultures transfected with SphK (SK-3T3) or vector alone (N-3T3) were photographed 12 days after transfection (40x magnification). **(B)** Colony formation in soft agar. N-3T3 and SK-3T3 cells were cultured in growth medium containing 0.33% agar and fed with the medium containing various concentrations of DMS every 2 days. Photographs were taken after 2 weeks of cultures followed by staining with MTT. **(C)** NIH 3T3 cells were transfected with V12-Ras or v-Src, SphK activity was measured after 48 h transfection. **(D)** Focus formation assays were performed in V 1 2-Ras, v-Src, or SphK transfected NIH 3T3 cells in the absence or presence of DMS (2.5 µM) over two weeks.

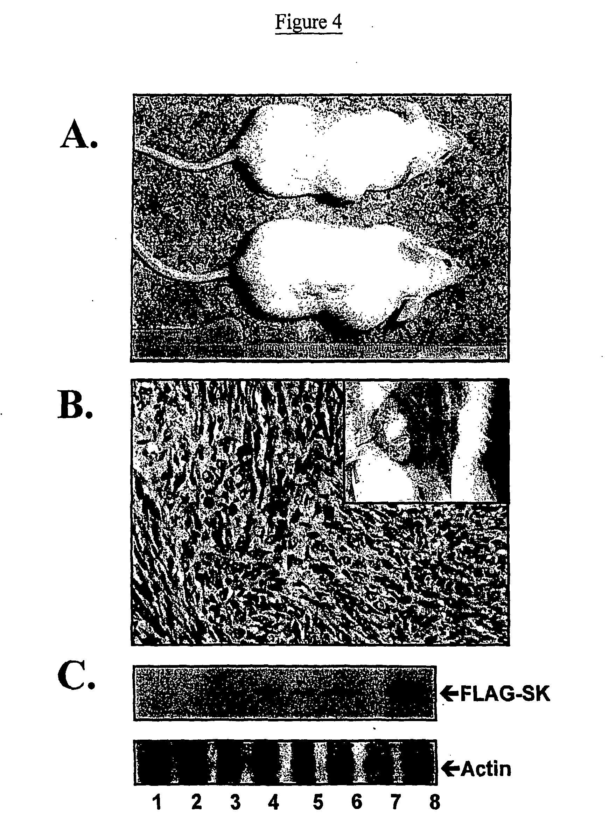

**Figure 4** is an image of cells overexpressing SphK are tumourigenic. **(A)** Photograph of tumours in the NOD/SCID mice injected with SphK-transfected NIH 3T3 cells. **(B)** Morphology of a tumour (insert) and the paraffin fixed section stained with hematoxalin and eosin (60x magnification). **(C)** Whole cell extracts from three individual tumours (lane 4-6) and their peripheral tissues (lane 1-3) and N-3T3 (lane 7) or SK-3T3 cells (lane 8) were analysed by Western blot. The top blot was probed with anti-FLAG antibody and the bottom with anti-actin antibody (Santa Cruz).

### DETAILED DESCRIPTION OF THE INVENTION

The present invention is predicated, in part, on the determination of a correlation between cell growth, in particular oncogenesis, and modulation in the level of activity of sphingosine kinase. The identification of this correlation permits the identification and rational design of methodology and products for use in therapy, prophylaxis and diagnosis of disease conditions characterised by aberrant, unwanted or otherwise inappropriate cell growth, in particular, uncontrolled oncogene induced proliferation.

Reference to "sphingosine kinase" should be understood as including reference to all forms of sphingosine kinase or derivative, homologue, analogue, equivalent or mimetic thereof or other molecules having the function of sphingosine kinase and to nucleic acid molecules encoding sphingosine kinase derivative, homologue, analogue, equivalent or mimetic thereof. This includes, for example, all protein or nucleic acid forms of sphingosine kinase or its functional derivative, homologue, analogue, equivalent or mimetic thereof including, for example, any isoforms which arise from alternative splicing of sphingosine kinase mRNA or mutants or polymorphic variants of sphingosine kinase. It should also be understood that reference to a "nucleic acid form of sphingosine kinase" is a reference to a nucleic acid encoding sphingosine kinase and includes reference to any sphingosine kinase regulatory element (such as promoters or enhancers) which regulate the expression of sphingosine kinase and includes regulatory elements which are located at a position other than between the sphingosine kinase genomic DNA transcription initiation and determination sites. "Sphingosine kinase" should also be understood to include reference to any other molecules which exhibit the functional activity of sphingosine kinase. Such molecules include, for example, endogenously expressed molecules which exhibit sphingosine kinase functional activity or molecules which have been introduced into the body and which mimic at least one of the sphingosine kinase functions. These molecules may be recombinant, synthetic or naturally occurring. To the extent that it is not specified, any reference to modulating the activity of sphingosine kinase includes modulating the expression of a nucleic acid molecule encoding sphingosine kinase or the functional activity of the sphingosine kinase expression product and should also be understood to include reference to modulating the expression or functional activity or a sphingosine kinase functional equivalent or derivative.

Reference to "modulating the functional activity" of sphingosine kinase should be understood as a reference to, down-regulating or otherwise altering any one or more of the functional activities or sphingosine kinase. This includes, for example, modulating the occurrence of one or more of the sphingosine kinase functional activities, modulating the rate at which a given activity is performed, modulating the level at which an activity is performed, modulating the number of activities which are capable of being performed or modulating the role or extent to which any activity is performed or the nature of an activity. Changes in the activity of sphingosine kinase can be effected by any one of a number of means including, post translational modification, associated proteins or other molecules or translation. Modulating said activity should also be understood to encompass decreasing the concentration levels of sphingosine kinase (for example by modulating expression of sphingosine kinase). Preferably, the subject functional activity is the level of activity of sphingosine kinase.

As detailed above, the present invention is predicated on the identification of a correlation between sphingosine kinase functional activity and cell growth, in particular, oncogenic cell proliferation. Without limiting the present invention to any one theory or mode of action, the inventors have found that the signalling cascade stimulated by the lipid kinase, sphingosine kinase, has a major role in oncogenesis. Specifically, constitutive activation of sphingosine kinase by overexpression in cells causes cell transformation and tumour formation, thereby indicating that a wild type human lipid kinase is by itself oncogenic. Furthermore, sphingosine kinase is also involved in Ras but not v-Src induced transformation. Finally inhibition of sphingosine kinase activity utilising a sphingosine kinase inhibitor not only reverses tranformation in cells overexpressing sphingosine kinase but does so also in Ras transformed cells. In this regard, reference to "modulating" the growth of a cell should be understood as a reference to down-regulating the growth of a cell. More specifically, reference to "down-regulating" should be understood as a reference to preventing, reducing or otherwise inhibiting one or more aspects of the growth of a cell (including inducing the apoptosis of or otherwise killing a cell). Reference to the "growth" of a cell should be understood in its broadest sense to include reference to all aspects of cell division/proliferation and/or differentiation. Preferably, the subject growth is proliferation.

Proliferation as disclosed herein is preferably uncontrolled proliferation such as that caused by the transformation of a cell. Said transformation is caused by the upregulation of an oncogene such as Ras or by sphingosine kinase overexpression oncogenic activity.

It should be understood that reference to a "cell" in the context of the present invention is a reference to any form or type of cell, irrespective of its origin. For example, the cell may be a naturally occurring normal or abnormal cell or it may be manipulated, modified or otherwise treated either *in vitro* or *in vivo* such as a cell which has been freezed/thawed or genetically, biochemically or otherwise modified either *in vitro* or *in vivo* (including, for example, cells which are the result of the fusion of two distinct cell types). In accordance with the invention, the cell is a neoplastic cell. By "neoplastic cell" is meant a cell exhibiting uncontrolled proliferation. The neoplastic cell may be a benign cell or a malignant cell. Preferably the cell is malignant. In one particular embodiment, the neoplastic cell is a malignant cell the proliferation of which would form a solid tumour such as a malignant cell derived from the colon, stomach, lung, brain, bone, oesophagus or pancreas.

A method of down-regulating the proliferation of a neoplastic cell which has become transformed by upregulation of the oncogene Ras is disclosed herein in which said method comprises contacting said neoplastic cell with an effective amount of an agent for a time and under conditions sufficient to inhibit sphingosine kinase.

Preferably the neoplastic cell is a malignant cell derived from the colon, stomach, lung, brain, bone, oesophagus, pancreas, breast, ovary or uterus.

In accordance with the invention said malignant cell has become transformed due to upregulation of the oncogene Ras.

Thus, in a first aspect the present invention provides an *in vitro* method of downregulating the proliferation of a neoplastic cell, wherein said neoplastic cell has become transformed by upregulation of the oncogene Ras, said method comprising contacting said cell with an effective amount of an agent for a time and under conditions sufficient to inhibit human sphingosine kinase.

It should be understood that the cell which is treated according to the method of the present invention may be located *ex vivo* or *in vivo*. By *"ex vivo*" is meant that the cell has been removed from the body of a subject wherein the modulation of its growth will be achieved *in vitro.* For example, the cell may be a non-neoplastic cell which is to be immortalised by up-regulating sphingosine kinase activity. Preferably, the cell is a neoplastic cell, such as a malignant cell, located *in vivo* (such as in the colon) and the down-regulation of its growth will be achieved by applying the method described herein *in vivo* to down-regulate the level of sphingosine kinase functional activity. It should also be understood that where reference is made to a specific cell type which is located in vivo, such as a malignant colorectal cell, this cell may be located in the colorectal area of the patient. If a colorectal primary malignancy has metastasised, the subject colorectal cell may be located in another region of the patient's body. For example, it may form part of a secondary tumour (metastasis) which is located, for example, in the liver, lymph node or bone.

In this regard, an "effective amount" means an amount necessary to at least partly attain the desired effect, or to delay the onset of, inhibit the progression of, or halt altogether, the onset of progression of a disease condition which is being treated. Such amounts will depend, of course, on the particular conditions being treated, the severity of the condition and individual patient parameters including age, physical conditions, size, weight and concurrent treatment. These factors are well known of those of ordinary skill in the art and can be addressed with no more than routine experimentation. It is preferred generally that a maximum dose be used, that is, the highest safe dose according to sound medical judgement. It will be understood by those of ordinary skill in the art, however, that a lower dose or tolerable dose may be administered for medical reasons, psychological reasons or for virtually any other reasons.

Modulation of the activity of sphingosine kinase by the administration of an agent to a cell can be achieved by one of several techniques including, introducing into said cell an agent (said agent being a proteinaceous or non-proteinaceous molecule) which directly or indirectly:
(i) modulates the expression of sphingosine kinase; or
(ii) modulates the functional activity of sphingosine kinase expression product.

In this regard, modulation of the functional activity of sphingosine kinase can be achieved by any one of several techniques, including, introducing into said cell a proteinaceous or non-proteinaceous molecule which directly or indireclty:
(i) modulates synthesis of said sphingosine kinase;
(ii) functions as an antagonist to said sphingosine kinase;

Said proteinaceous molecule may be derived from natural, recombinant or synthetic sources including fusion proteins or following, for example, natural product screening. Said non-proteinaceous molecule may be derived from natural sources, such as for example natural product screening or may be chemically synthesised. The present invention contemplates use of chemical analogs of said sphingosine kinase capable of acting as agonists or antagonists of said sphingosine kinase. Chemical agonists may not necessarily be derived from said sphingosine kinase but may share certain conformational similarities. Alternatively, chemical agonists may be specifically designed to mimic certain physiochemical properties of said sphingosine kinase. Antagonists may be any compound capable of blocking, inhibiting or otherwise preventing said sphingosine kinase from carrying out its normal biological functions (for example N,N-dimethylsphingosine or DL-threo-dihydrosphingosine). Antagonists include monoclonal antibodies specific for said sphingosine kinase, or parts of said sphingosine kinase, and antisense nucleic acids which prevent transcription or translation of genes or mRNA in the subject cells. Modulation of expression may also be achieved utilising antigens, RNA, ribosomes, DNAzymes, RNA aptamers, antibodies or molecules suitable for use in co-suppression.

Said proteinaceous or non-proteinaceous molecule may act either directly or indirectly to modulate the expression of sphingosine kinase or the activity of the sphingosine kinase expression product. Said molecule acts directly if it associates with the sphingosine kinase nucleic acid molecule or expression product to modulate expression or activity. Said molecule acts indirectly if it associates with a molecule other than the sphingosine kinase nucleic acid molecule or expression product which other molecule either directly or indirectly modulates the expression or activity of the sphingosine kinase nucleic acid molecule or expression product. Accordingly, the method of the present invention encompasses regulation of sphingosine kinase nucleic acid molecule expression or expression product functional activity via the induction of a cascade of regulatory steps.

Still without limiting the operation of the present invention to any one theory or mode of action, sphingosine kinase is known to function via a signalling pathway which is commonly referred to as the sphingosine kinase signalling pathway. The "sphingosine kinase signalling pathway" is defined as a signalling pathway which utilises sphingosine kinase. In terms of indirect modulation of sphingosine kinase functional activity, it should be understood that the object of the invention could be achieved by modulating the activity of sphingosine kinase signalling pathway components which function either upstream or downstream of sphingosine kinase, to the extent that it forms part of this pathway. For example, modulation of said "sphingosine kinase activity" may be achieved by:
(i) modulation of the catalytic activity of sphingosine kinase by competition with substrate (for example, sphingosine or ATP);
(ii) interference with the catalytic activity of sphingosine kinase by an allosteric mechanisms (binding to sites on the molecule other than the substrate-binding sites); or
(iii) interfering with enzyme activation, such as by altering:
   - post-translational covalent modification such as phosphorylation, lipid modification
   - non-covalent coupling to a required co-activator such as a protein, lipid or ion
   - subcellular localisation of the enzyme.

"Derivatives" include fragments, parts, portions, mutants, variants and mimetics from natural, synthetic or recombinant sources including fusion proteins. Parts or fragments include, for example, active regions of sphingosine kinase. Derivatives may be derived from insertion, deletion or substitution of amino acids. Amino acid insertional derivatives include amino and/or carboxylic terminal fusions as well as intrasequence insertions of single or multiple amino acids. Insertional amino acid sequence variants are those in which one or more amino acid residues are introduced into a predetermined site in the protein although random insertion is also possible with suitable screening of the resulting product. Deletional variants are characterized by the removal of one or more amino acids from the sequence. Substitutional amino acid variants are those in which at least one residue in the sequence has been removed and a different residue inserted in its place. An example of substitutional amino acid variants are conservative amino acid substitutions. Conservative amino acid substitutions typically include substitutions within the following groups: glycine and alanine; valine, isoleucine and leucine; aspartic acid and glutamic acid; asparagine and glutamine; serine and threonine; lysine and arginine; and phenylalanine and tyrosine. Additions to amino acid sequences include fusions with other peptides, polypeptides or proteins.

Reference to "homologues" should be understood as a reference to sphingosine kinase nucleic acid molecules or proteins derived from species other than the species being treated.

Chemical and functional equivalents of sphingosine kinase nucleic acid or protein molecules should be understood as molecules exhibiting any one or more of the functional activities of these molecules and may be derived from any source such as being chemically synthesized or identified via screening processes such as natural product screening.

The derivatives include fragments having particular epitopes or parts of the entire protein fused to peptides, polypeptides or other proteinaceous or non-proteinaceous molecules.

Analogues contemplated herein include modification to side chains, incorporating of unnatural amino acids and/or their derivatives during peptide, polypeptide or protein synthesis and the use of crosslinkers and other methods which impose conformational constraints on the proteinaceous molecules or their analogues.

Derivatives of nucleic acid sequences may similarly be derived from single or multiple nucleotide substitutions, deletions and/or additions including fusion with other nucleic acid molecules. The derivatives of the nucleic acid molecules described herein include oligonucleotides, PCR primers, antisense molecules, molecules suitable for use in cosuppression and fusion of nucleic acid molecules. Derivatives of nucleic acid sequences also include degenerate variants.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carboethoxylation with diethylpyrocarbonate.

Examples of incorporating unnatural amino acids and derivatives during protein synthesis include, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acids contemplated herein is shown in Table 1.

**TABLE 1**

| Non-conventional amino acid | Code | Non-conventional amino acid | Code |
|---|---|---|---|
| α-aminobutyric acid | Abu | L-N-methylalanine | Nmala |
| α-amino-α-methylbutyrate | Mgabu | L-N-methylarginine | Nmarg |
| aminocyclopropane-carboxylate | Cpro | L-N-methylasparagine | Nmasn |
| | | L-N-methylaspartic acid | Nmasp |
| aminoisobutyric acid | Aib | L-N-methylcysteine | Nmcys |
| aminonorbornyl-carboxylate | Norb | L-N-methylglutamine | Nmgln |
| | | L-N-methylglutamic acid | Nmglu |
| cyclohexylalanine | Chexa | L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl- -aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Norn |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmorn | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Tbug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Mom |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1-carboxy-1-(2,2-diphenyl- Nmbc | | | |
| ethylamino)cyclopropane | | | |

Crosslinkers can be used, for example, to stabilise 3D conformations, using homobifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety.

The molecules which may be administered to a mammal in accordance with the present invention may also be linked to a targeting means such as a monoclonal antibody, which provides specific delivery of these molecules to the target cells.

A further aspect of the present invention relates to the use of the invention in relation to the treatment and/or prophylaxis of disease conditions. Without limiting the present invention to any one theory or mode of action, the inventors have determined not only that constitutive activation of sphingosine kinase causes cell transformation and tumour development, thereby indicating that sphingosine kinase is by itself oncogenic, but that sphingosine kinase inhibition is also effective in down-regulating neoplastic cell proliferation where the subject cell has been transformed by certain unrelated oncogenes such as Ras induced transformation. Accordingly, the method of the present invention is for use in the treatment of primary and secondary malignancies such as those associated with solid tumours of the colon, stomach, lung, brain, bone, oesophagus and pancreas which arise from the proliferation of Ras transformed cells.

Accordingly, a method for the treatment and/or prophylaxis of a condition characterised by aberrant, unwanted or otherwise inappropriate cell growth in a mammal, is described herein in which said method comprises administering to said mammal an effective amount of an agent for a time and under conditions sufficient to modulate the functional activity of sphingosine kinase wherein down-regulation of the functional activity of said sphingosine kinase down-regulates the subject cell growth.

Reference to "aberrant, unwanted or otherwise inappropriate" cell growth should be understood as a reference to over active cell growth, to physiologically normal cell growth which is inappropriate in that it is unwanted or to insufficient cell growth. Preferably, said inappropriate cell growth is uncontrolled cell proliferation.

In accordance with the invention said uncontrolled cell proliferation is caused by the transformation of the cell by Ras oncogene up-regulation.

Thus a further aspect of the invention provides an agent capable of inhibiting human sphingosine kinase for use in the treatment of a neoplastic condition in a human wherein said neoplasia has become transformed by upregulation of the oncogene Ras.

Still more preferably said cell is a malignant cell which forms a solid tumour of the colon, stomach, lung, brain, bone, oesophagus, pancreas, breast, ovary or uterus.

Preferably, said agent is N,N-dimethylspingosine or DL-threo-dihydrophingosine.

The method of the present invention preferably facilitates the subject proliferation being reduced, retarded or otherwise inhibited. Reference to "reduced, retarded or otherwise inhibited" should be understood as a reference to inducing or facilitating the partial or complete inhibition of cell proliferation. Said inhibition may occur by either direct or indirect mechanisms and includes the induction of cellular apoptosis or other cellular killing mechanisms.

The subject of the treatment or prophylaxis is generally a mammal such as but not limited to human, primate, liverstock animal (eg. sheep, cow, horse, donkey, pig), companion animal (eg. dog, cat), laboratory test animal (eg. mouse, rabbit, rat, guinea pig, hamster), captive wild animal (eg. fox, deer). Preferably the mammal is a human or primate. Most preferably the mammal is a human. Although the present invention is exemplified utilising a murine model, this is not intended as a limitation on the application of the method of the present invention to other species, in particular, humans.

Preferably said neoplastic condition is a malignant condition and even more preferably a solid malignancy such as a tumour of the colon, stomach, lung, brain, bone, oesophagus or pancreas.

In accordance with the invention the subject malignancy is caused by transformation of the cell via Ras oncogene up-regulation.

Still more preferably said agent is N,N-dimethylsphingosine or DL-threo-dihydrophingosine.

Reference herein to "treatment" and "prophylaxis" is to be considered in its broadest context. The term "treatment" does not necessarily imply that a mammal is treated until total recovery. Similarly, "prophylaxis" does not necessarily mean that the subject will not eventually contract a disease condition. Accordingly, treatment and prophylaxis including amelioration of the symptoms of a particular condition or preventing or otherwise reducing the risk of developing a particular condition. The term "prophylaxis" may be considered as reducing the severity or onset of a particular condition. "Treatment" may also reduce the severity of an existing condition.

Administration of the agent (including sphingosine kinase or functional equivalent, derivative, homologue, analogue or mimetic thereof or sphingosine kinase nucleic acid molecule) [herein referred to as "modulatory agent"], in the form of a pharmaceutical composition, may be performed by any convenient means. The modulatory agent of the pharmaceutical composition is contemplated to exhibit therapeutic activity when administered in an amount which depends on the particular case. The variation depends, for example, on the human or animal and the modulatory agent chosen. A broad range of doses may be applicable. Considering a patient, for example, from about 0.1 mg to about 1 mg of modulatory agent may be administered per kilogram of body weight per day. Dosage regimes may be adjusted to provide the optimum therapeutic response. For example, several divided doses may be administered daily, weekly, monthly or other suitable time intervals or the dose may be proportionally reduced as indicated by the exigencies of the situation. The modulatory agent may be administered in a convenient manner such as by the oral, intravenous (where water soluble), intraperitoneal, intramuscular, subcutaneous, intradermal or suppository routes or implanting (e.g. using slow release molecules). The modulatory agent may be administered in the form of pharmaceutically acceptable nontoxic salts, such as acid addition salts or metal complexes, e.g. with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. If the active ingredient is to be administered in tablet form, the tablet may contain a binder such as tragacanth, corn starch or gelatin; a disintegrating agent, such as alginic acid; and a lubricant, such as magnesium stearate.

Routes of administration include, but are not limited to, respiratorally, intratracheally, nasopharyngeally, intravenously, intraperitoneally, subcutaneously, intracranially, intradermally, intramuscularly, intraoccularly, intrathecally, intracereberally, intranasally, infusion, orally, rectally, via IV drip patch and implant.

In another aspect the present invention relates to use of an agent capable of inhibiting human sphingosine kinase in the manufacture of a medicament for the treatment of a neoplastic condition in a human wherein said neoplasia has become transformed by upregulation of the oncogene Ras, wherein said agent, inhibition or condition are as defined herein.

Pharmaceutical compositions comprising a modulatory agent as hereinbefore defined and one or more pharmaceutically acceptable carriers and/or diluents may be used in the methods and uses described herein. Said modulatory agents are referred to as the active ingredients.

The pharmaceutical forms suitable for injectable use include sterile aqueous solutions (where water soluble) or dispersions and sterile powders for the extemporaneous preparation of sterile injectable solutions or dispersion or may be in the form of a cream or other form suitable for topical application. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of superfactants. The preventions of the action of microorganisms can be brought about by various antibacterial and antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilisation. Generally, dispersions are prepared by incorporating the various sterilised active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions in such that a suitable dosage will be obtained. Preferred compositions or preparations according to the present invention are prepared so that an oral dosage unit form contains between about 0.1 µg and 2000 mg of active compound.

The tablets, troches, pills, capsules and the like may also contain the components as listed hereafter: a binder such as gum, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations. The pharmaceutical composition may also comprise genetic molecules such as a vector capable of transfecting target cells where the vector carries a nucleic acid molecule encoding a modulatory agent. The vector may, for example, be a viral vector.

Screening for the modulatory agents hereinbefore defined can be achieved by any one of several suitable methods known to those of skill in the art including, but in no way limited to, contacting a cell comprising the sphingosine kinase gene or functional equivalent or derivative thereof with an agent and screening for the modulation of sphingosine kinase protein production or functional activity, modulation of the expression of a nucleic acid molecule encoding sphingosine kinase or modulation of the activity or expression of a downstream sphingosine kinase cellular target. Detecting such modulation can be achieved utilising techniques such as Western blotting, electrophoretic mobility shift assays and/or the readout of reporters of sphingosine kinase activity.

It should be understood that the sphingosine kinase gene or functional equivalent or derivative thereof may be naturally occurring in the cell which is the subject of testing or it may have been transfected into a host cell for the purpose of testing. Further, the naturally occurring or transfected gene may be constitutively expressed - thereby providing a model useful for, inter alia, screening for agents which down regulate sphingosine kinase activity, at either the nucleic acid or expression product levels, or the gene may require activation - thereby providing a model useful for, inter alia, screening for agents which up regulate sphingosine kinase expression. Further, to the extent that a sphingosine kinase nucleic acid molecule is transfected into a cell, that molecule may comprise the entire sphingosine kinase gene or it may merely comprise a portion of the gene such as the portion which regulates expression of the sphingosine kinase product. For example, the sphingosine kinase promoter region may be transfected into the cell which is the subject of testing. In this regard, where only the promoter is utilised, detecting modulation of the activity of the promoter can be achieved, for example, by ligating the promoter to a reporter gene. For example, the promoter may be ligated to luciferase or a CAT reporter, the modulation of expression of which gene can be detected via modulation of fluorescence intensity or CAT reporter activity, respectively.

In another example, the subject of detection could be a downstream sphingosine kinase regulatory target, rather than sphingosine kinase itself. Such detection may be achieved utilising technologies such as microarrays (e.g. Chip arrays, nylon arrays), SAGE analysis, RDA or Differential Display.

These methods provide a mechanism for performing high throughput screening of putative modulatory agents such as the proteinaceous or non-proteinaceous agents comprising synthetic, combinatorial, chemical and natural libraries. These methods will also facilitate the detection of agents which bind either the sphingosine kinase nucleic acid molecule or expression product itself or which modulate the expression of an upstream molecule, which upstream molecule subsequently modulates sphingosine kinase expression or expression product activity. Accordingly, these methods provide a mechanism of detecting agents which either directly or indirectly modulate sphingosine kinase expression and/or activity.

As detailed earlier, reference to "sphingosine kinase" should be understood as a reference to either the sphingosine kinase expression product or to a nucleic acid molecule encoding sphingosine kinase. It should also be understood as a reference to a portion or fragment of the sphingosine kinase molecule such as the regulatory region of the sphingosine kinase nucleic acid molecule. Alternatively, the molecule may comprise the binding/active portion of the expression product. In this regard, the sphingosine kinase nucleic acid molecule and/or expression product is expressed in a cell. The cell may be a host cell which has been transfected with the sphingosine kinase nucleic acid molecule or it may be a cell which naturally contains the sphingosine kinase gene.

The screening method herein defined may be based on detecting an "altered expression phenotype associated with said sphingosine kinase". This should be understood as the detection of cellular or cell culture condition changes associated with modulation of the activity of sphingosine kinase. These may be detectable for example as intracellular changes or changes observable extracellularly. For example, this includes, but is not limited to, detecting changes in expression product levels, cell culture condition changes or, to the extent that the sphingosine kinase regulatory region is ligated to a reporter molecule such as luciferase or CAT, detecting changes in reporter molecule expression. Alternatively, this screening system may be established to detect changes in the expression of downstream molecules which are regulated by the sphingosine kinase expression product.

Diagnostic methodology based on screening individuals for the presence of sphingosine kinase or mRNA or protein or the specific forms of sphingosine kinase which are transcribed and/or translated by a given population of cells is also disclosed. The screening methodology may be directed to qualitative and/or quantitative sphingosine kinase analysis. This is particularly useful, for example, for determining whether a given individual is predisposed to or resistant to diseases/disorders in which aberrant, unwanted or otherwise inappropriate cell growth is a component of the disease state and/or is predisposed or resistant to the development of certain forms of aberrant, unwanted or otherwise inappropriate cell growth. Such screening can be performed utilising methods which would be known to those skilled in the art including, but not limited to:
(i) The use of sphingosine kinase assays to screen for altered sphingosine kinase activity or levels. These parameters can be screened for either in the bodily fluids or tissues of individuals. Although sphingosine kinase is generally not secreted, its down-stream product sphingosine kinase-1-phosphate is secreted. Modulation of the levels of this molecule could therefore be used as an indicator of changes in sphingosine kinase levels or activity. Nevertheless, screening for extracellular sphingosine kinase should not be excluded.
(ii) Analysis of sphingosine kinase for possible mutations/polymorphisms (SNPs) and mutations can be performed by melting curve analysis of PCR generated DNA using the LightCycler system (Roche).
(iii) The screening of SNPs involving sphingosine kinase using Chip Arrays (e.g. Affimetrix GeneChipSNP mapping or using the Incyte technology plaform *fSSCP* for SNP discovery.

The present invention is further described by the following examples.

### EXAMPLE 1

### TRANSFECTION AND ANALYSIS OF NIH 3T3 FIBROBLASTS

To investigate the oncogenic role of SphK, the nontransformed NIH 3T3 fibroblasts were transfected with human SphK cDNA that was recently cloned in our laboratory (Piston *et al.,* 2000). Pooled stable transfectants (referred as SK-3T3) were used to avoid the phenotypic artifacts that may due to the selection and propagation of individual clones from single transfected cells. In the SK-3T3 cell pools SphK activity was increased by over 600-fold (Fig. 1a) in comparison with the empty vector-transfected NIH 3T3 cells (*N-*3T3). Immunoblotting analysis showed a specific protein band with an apparent molecular weight consistent with the predicted size of FLAG-tagged human SphK that was detected only in the SK-3T3 cell pools but absent in N-3T3 cells (Fig. 1c). Intracellular levels of S1P, the direct product of SphK, were also increased in SK-3T3 cells by 4- to 5-fold, indicating the stable transfectants with constitutively activation of SphK (Fig. 1b). S1P levels were not directly proportional to the increase in SphK activity assayed *in vitro* perhaps due to rapid degradation of S1P by S1P phosphatase and S1P lyase and the limited availability of sphingosine as a substrate for S1P formation.

Growth curves showed a significant difference between the SK-3T3 cell pools and the controls (Fig. 2a). Stable expression of SphK dramatically enhanced cell growth in the media containing either 1% or 10% serum. Even in serum-free medium for up to 7 days, SK-3T3 cells survived and grew whilst the N-3T3 cells underwent death. Furthermore, when the cells reached saturation density SK-3T3 cells continued to proliferate (Fig. 2b) suggesting an escape from contact inhibition. Treatment of SK-3T3 cells with a specific inhibitor of SphK, *N,N*-dimethylsphingosine (DMS), significantly diminished the enhanced proliferation induced by overexpression of SphK, whilst DMS had no effect on proliferation of N-3T3 cells (Fig. 2c). These results indicate that the constitutive activation of SphK in the stably SphK-transfected cells reduces two key growth limiting properties: serum dependence and contact inhibition.

The transforming activity assayed by focus formation in NIH 3T3 cells showed that cells transfected with SphK but not empty vector induced numerous foci (Table 1 and Fig. 3 a). Both SphK and control vectors displayed similar efficiency in the generation of G418-resistant colonies indicating that the transforming activity was not due to non-specific effect of transfection. Furthermore, SK-3T3 cells formed vigorous colonies in soft agar (Table 2 and Fig. 3b) revealing the acquisition of anchorage-independent growth. Although N-3T3 cells exhibited a background level of colony formation that may due to spontaneous transformation, overexpression of SphK resulted in a 20∼50-fold increase in the number of colonies and an obvious increase in colony size (Table 2). Importantly, DMS inhibited the transforming capacity of SphK in a dose-dependent manner with 2.5 µM DMS resulting in total reversion to the normal phenotype (Fig. 3b). This suggested that the increased activity of SphK rather than its mere overexpression is responsible for the transforming capacity of this enzyme.

### EXAMPLE 2

### TRANSFECTION AND ANALYSIS OF ONCOGENE TRANSFORMED CELLS

When NIH 3T3 cells were transfected with an activated mutant Ras (V12-Ras), SphK activity was significantly increased by 178±22% in comparison to the parent cells (Fig. 3c). By contrast the cells transfected with v-Src (Fig. 3c) or dominant-negative Ras (N17-Ras had no changes in the activity of SphK, suggesting a specific involvement of SphK in some oncogenic transformation. Moreover, when the cells were treated with DMS, the focus formation was reduced by 42±4% in V12-Ras transformed cells but there were no changes in v-Src transformed cells (Fig. 3d), indicating an important role of SphK in Ras transformation. The likelihood that there are SphK independent pathways operating is suggested by the partial effect of DMS at the dose (2.5 µM) that was fully effective in SphK transformed cells (Fig 3d). On the other hand, the inability of DMS to inhibit v-Src transformation rules out non-specific effects of DMS or a general toxicity resulting from inhibition of SphK. The increased SphK activity thereby exerts a transforming potential not only in its own right by overexpression but is also involved in oncogenic transformation, eg., induced by Ras.

### EXAMPLE 3

### ANALYSIS OF TUMORIGENICITY

Tumorigenicity was then directly tested in NIH 3T3 cells overexpressing SphK. When the SphK-transfected NIH 3T3 cells from either the stable transfectant pools or selected clones were injected subcutaneously into NOD/SCID mice, tumours became apparent at the site of injection within 3 to 4 weeks (Table 4 and Fig. 4). No mice injected with the vector-transfected 3T3 cells induced tumours during 10-weeks of observation. Histological appearances of tumour sections displayed the morphologies of fibrosarcoma with many mitotic figures (Fig. 4). Western blot analysis of extracts derived from tumours showed high levels of FLAG-tagged protein (Fig. 4), revealing that the neoplastic cells retain and express the SphK transgenes. Thus, the tumours were developed from the injected SphK-transfected cells but not from spontaneously transformed NIH 3T3. This is the first demonstration that a wild type lipid kinase gene, human SphK, acts as an oncogene providing a potential linkage between this enzyme and mammalian tumour pathogenesis. Thus, our finding extends the understanding of phospholipids, particularly sphingolipids, as signal transducers regulating cellular growth, transformation and oncogenesis.

### EXAMPLE 4

### MATERIALS AND METHODS

### (a) Sphingosine kinase transfection

NIH 3T3 fibroblasts were obtained from the American Type Culture Collection and maintained in DMEM (GIBCO BRL), supplemented with 10% calf serum. The human SphK cDNA tagged with a FLAG tag was subcloned into the pcDNA3 plasmid (Invitrogen Corp.) as previous described (Pitson *et al.,* 2000). For transient transfections, 5 µg of plasmids were transfected to 5 x 10⁵ using Lipofectamine Plus (GIBCO BRL) according to the manufacturer's protocols. For stable expression, the calcium phosphate precipitation method was used and the transfectants were selected in medium containing 500 µg/ml G418 (GIBCO BRL). The nonclonal pools of G418-resistant transfected cells were collected andused to avoid clonal variability. For some experiments, the selected clones of stable transfectants were used.

### (b) Measurement of sphingosine kinase activity

SphK activity was measured by incubating the cytosolic fraction with 10 µM sphingosine dissolved in 5% Triton X-100 and [γ³²P]ATP (1mM, 0.5mCi/ml) for 15 min at 37°C as described previously (Xia *et al.,* 1999). For assay of intracellular level of S1P, cells were labelled with [³H]sphingosine (1 µM, 2 µCi/ml) for 30 min and radioactivity incorporated into cellular lipids were extracted. [³H]S1P was then resolved on TLC with 1-butanol/methanol/acetic acid/water (8:2:1:2, vol/vol), visualized and quantified by Phosphoimager®.

### (c) Culture of sphingosine kinase-3T3 cells

Stably transfected NIH 3T3 cells were plated in 48-well plates (1,000 cells per well) in DMEM containing 10% calf serum. After 8 h, cells were washed twice with DMEM and then grown in DMEM containing 1 or 10% serum and serum-free medium (DMEM containing 0.1 % BSA). At the indicated times, cells were incubated with 1 mg/ml MTT for 4 h. The formazan product was solubilized by 10% in SDS in 10 mM HCl and assessed by spectrophotometry at 570 nm and 650 nm absorbance. In some experiments, cells were trypsinzed and counted in a hemocytometer.

### (d) Focus formation assay

For focus formation assay, low passage NIH 3T3 cells were transfected with the activated V12 Ras, v-Src (gifts of Dr. Julian Downward(, SphK, or empty vector, respectively, using Lipofectamine Plus as described in (a) above. Two days later, the transfected cells were split to 6-well plates. After reaching confluence, they were kept for two weeks in DMEM containing 5% calf serum. The foci were visualized and scored after stained with 0.5% crystal violet. For soft agar assay, suspensions of 1 x 10⁴ cells from the stable transfected pools in a growth medium containing 0.33% agar were overlaid onto 0.6% agar gel in the absence or presence of DMS at various concentrations. After a 14-day incubation colonies were stained with 0.1 mg/ml MTT and that over 0.1 mm in diameter were scored as positive.

### (e) Tumour induction

NOD/SCID mice were bred and maintained under sterile conditions. Four- to six-week-old mice were injected subcutaneously with 5x10⁵ cells in 200 µl sterile PBS from various cell lines (stable SphK- and vector-transfected NIH 3T3 cell pools, two colonies of SphK transfectants). Each cell line was tested in 3 different animals.

**Table 2. Transformation assays in transfected NIH 3T3 cells.**

| | Focus formation | Colonies in soft agar | |
|---|---|---|---|
| Cell Line | Number | Number | Size (mm) |
| N-3T3 | 1.7±1.5 | 3.3±2.1 | <0.1 |
| SK-3T3 | 65.7±9.6 | 122.3±17.6 | 0.1∼0.45 |

Focux formation was assayed in NIH 3T3 cells transiently transfected with SphK (SK-3T3) or vector (N-3T3). Transfected cells were plated in 6-well plates and cultured for 2∼3 weeks prior to crystal violet staining. Colony formation in soft agar was determined in the stable transfected cells. Results shown are the mean ± SD from 3∼5 experiments done in duplicate or triplicate.

**Table 3. Tumourigenesis in NOD/SCID mice.**

| Cell lines | Tumours/ injections | Tumour size | |
|---|---|---|---|
| | | cm | cm³ |
| N-3T3 | 0/3 | 0 | |
| SK-3T3 | 3/3 | 2.8 x 1.8 x 1.1 | 5.54 |
| stable pools | | 1.9 x 1.5 x 1.0 | 2.85 |
| | | 1.1 x 0.9 x 0.8 | 0.79 |
| Clone KT-2 | 3/3 | 3.2 x 1.8. x 1.2 | 6.91 |
| | | 1.6 x 1.2 x 0.5 | 0.96 |
| | | 2.5 x 1.6 x 1.3 | 5.20 |
| Clone KT-5 | 3/3 | 2.7 x 1.6 x 1.2 | 5.18 |
| | | 2.2 x 1.5 x 1.1 | 3.63 |
| | | 1.8 x 1.7 x 0.9 | 2.75 |

NOD/SCID mice were injected with cells (5x10⁵ cells per mouse) from vector- or SphK-transfected NIH 3T3 cell pools (N-3T3 or SK-3T3), or two individual SphK-transfected clones (KT-2 and KT-5). Tumour size was determined 4 weeks after injection.

### BIBLIOGRAPHY

Allessenko, A.V., (1998) Biochemistry (Mosc) 63:62-68.
Culliver, O., Pirianov, G., Kleuser, B., Vanek, P.G., Coso, O.A., Gutkind, J.S. and Spiegel, S. (1996) Nature 381: 800-803.
Igarashi, Y. (1997) J. Biochem. 122:1080-1087.
Meyer zu Heringdorf, D., van Koppen, C.J. and Jakobs, K.H. (1997) FEBS Lett 410: 34-38.
Pitson, S.M. et al. (2000) Biochem. J. submitted.
Spiegel, S., Culliver, O., Edsall, L., Kohama, T., Menzeleev, R.; Olivera, A., Thomas, D., Tu, Z., Van Brocklyn, J. and Wang, F. (1998) Biochemistry (Mosc) 63:69-73.
Xia, P., Gamble, J.R., Rye, K.-A., Wang, L., Hii, C.S.T., Cockerill, P., Khew-Goodall, Y., Bert, A.G., Barter, P.J., and Vadas, M.A. (1998) Proc. Natl. Acad, Sci. USA 95:14196-14201.
Xia, P., Vadas, M.A., Rye, K.A., Barter, P.J., Gamble, J.R. (1999) J. Biol. Chem. 274:33143-33147.

## Claims

1. An agent capable of inhibiting human sphingosine kinase for use in the treatment of a neoplastic condition in a human wherein said neoplasia has become transformed by upregulation of the oncogene Ras.

2. An agent according to claim 1, wherein said neoplastic condition is a malignant condition.

3. An agent according to claim 2, wherein said malignant condition is a solid tumour of the colon, stomach, lung, brain, bone, oesophagus, pancreas, breast, ovary or uterus.

4. An agent according to any one of claims 1 to 3, wherein said inhibition is achieved by contacting neoplastic cells of said neoplastic condition with a proteinaceous or non-proteinaceous molecule which functions as an antagonist to the sphingosine kinase expression product.

5. An agent according to claim 4, wherein said antagonist is a sphingosine kinase substrate competitor.

6. An agent according to claim 5, wherein said substrate is ATP.

7. An agent according to claim 5, wherein said substrate is sphingosine.

8. An agent according to claim 7, wherein said antagonist is N,N-dimethylsphingosine.

9. An agent according to claim 7, wherein said antagonist is DL-threo-dihydrosphingosine.

10. An agent according to claim 4, wherein said antagonist downregulates sphingosine kinase enzyme activation. -

11. An *in vitro* method of downregulating the proliferation of a neoplastic cell, wherein said neoplastic cell has become transformed by upregulation of the oncogene Ras, said method comprising contacting said cell with an effective amount of an agent for a time and under conditions sufficient to inhibit human sphingosine kinase.

12. A method according to claim 11, wherein said cell is a malignant cell.

13. The method according to claim 12, wherein said malignant cell is a cell from the colon, stomach, lung, brain, bone, oesophagus, pancreas, breast, ovary or uterus.

14. The method according to any one of claims 11 to 13, wherein said agent is as defined in any one of claims 4 to 10.

15. Use of an agent capable of inhibiting human sphingosine kinase in the manufacture of a medicament for the treatment of a neoplastic condition in a human wherein said neoplasia has become transformed by upregulation of the oncogene Ras, wherein said agent, inhibition or condition are as defined in any one of claims 2 to 10.

## Patentansprüche

1. Mittel, das dazu fähig ist, die humane Sphingosinkinase zu inhibieren, zur Verwendung bei der Behandlung eines neoplastischen Zustands in einem Menschen, wobei die Neoplasie durch die Hochregulation des Onkogens Ras transformiert worden ist.

2. Mittel nach Anspruch 1, wobei der neoplastische Zustand ein maligner Zustand ist.

3. Mittel nach Anspruch 2, wobei der maligne Zustand ein solider Tumor des Colons, des Magens, der Lunge, des Gehirns, des Knochens, des Oesophagus, des Pankreas, der Brust, des Ovars oder des Uterus ist.

4. Mittel nach einem der Ansprüche 1 bis 3, wobei die Inhibition durch Inkontaktbringen von neoplastischen Zellen des neoplastischen Zustands mit einem proteinartigen oder nicht proteinartigen Molekül, das als Antagonist für das Expressionsprodukt der Spingosinkinase wirkt, erreicht wird.

5. Mittel nach Anspruch 4, wobei der Antagonist ein Spingosinkinase-Substratkonkurrent ist.

6. Mittel nach Anspruch 5, wobei das Substrat ATP ist.

7. Mittel nach Anspruch 5, wobei das Substrat Sphingosin ist.

8. Mittel nach Anspruch 7, wobei der Antagonist N,N-Dimethylsphingosin ist.

9. Mittel nach Anspruch 7, wobei der Antagonist D,L-threo-Dihydrosphingosin ist.

10. Mittel nach Anspruch 4, wobei der Antagonist die Enzymaktivierung der Sphingosinkinase herabreguliert.

11. *In vitro*-Verfahren zur Herabregulation der Proliferation einer neoplastischen Zelle, wobei die neoplastische Zellheraufregulierte durch Hochregulation des Onkogens Ras transformiert worden ist, wobei das Verfahren das Inkontaktbringen der Zelle mit einer wirksamen Menge eines Mittels für eine Zeit und unter Bedingungen, die ausreichen, um die humane Sphingosinkinase zu inhibieren, umfasst.

12. Verfahren nach Anspruch 11, wobei die Zelle eine maligne Zelle ist.

13. Verfahren nach Anspruch 12, wobei die maligne Zelle eine Zelle aus dem Colon, dem Magen, der Lunge, dem Gehirn, dem Knochen, dem Oesophagus, dem Pankreas, der Brust, dem Ovar oder dem Uterus ist.

14. Verfahren nach einem der Ansprüche 11 bis 13, wobei das Mittel, wie in einem der Ansprüche 4 bis 10 definiert, ist.

15. Verwendung eines Mittels, das dazu fähig ist, die humane Sphingosinkinase zu inhibieren, zur Herstellung eines Medikaments zur Behandlung eines neoplastischen Zustands in einem Menschen, wobei die Neoplasie durch die Hochregulation des Onkogens Ras transformiert worden ist, wobei das Mittel, die Inhibition oder der Zustand, wie in einem der Ansprüche 2 bis 10 definiert, sind.

## Revendications

1. Agent capable d'inhiber la sphingosine kinase humaine pour utilisation dans le traitement d'un état néoplasique chez un humain dans lequel ladite néoplasie a été transformée par surexpression de l'oncogène Ras.

2. Agent selon la revendication 1, dans lequel ledit état néoplasique est un état malin.

3. Agent selon la revendication 2 , dans lequel ledit état malin est une tumeur solide du côlon, de l'estomac, du poumon, du cerveau, des os, de l'oesophage, du pancréas, du sein, de l'ovaire ou de l'utérus.

4. Agent selon l'une quelconque des revendications 1 à 3, dans lequel ladite inhibition est obtenue en mettant en contact des cellules néoplasiques dudit état néoplasique avec une molécule protéique ou non protéique qui fonctionne comme un antagoniste du produit d'expression de la sphingosine kinase.

5. Agent selon la revendication 4, dans lequel ledit antagoniste est un substrat compétiteur de la sphingosine kinase.

6. Agent selon la revendication 5, dans lequel ledit substrat est l'ATP.

7. Agent selon la revendication 5, dans lequel ledit substrat est la sphingosine.

8. Agent selon la revendication 7, dans lequel ledit antagoniste est la N,N-diméthylsphingosine.

9. Agent selon la revendication 7, dans lequel ledit antagoniste est la DL-thréo-dihydrosphingosine.

10. Agent selon la revendication 4, dans lequel ledit antagoniste réprime l'activation de l'enzyme sphingosine kinase.

11. Méthode *in vitro* de répression de la prolifération d'une cellule néoplasique, dans laquelle ladite cellule néoplasique a été transformée par surexpression de l'oncogène Ras, ladite méthode comprenant la mise en contact de ladite cellule avec une quantité efficace d'un agent pendant un temps et dans des conditions suffisants pour inhiber la sphingosine kinase humaine.

12. Méthode selon la revendication 11, dans laquelle ladite cellule est une cellule maligne.

13. Méthode selon la revendication 12 , dans laquelle ladite cellule maligne est une cellule du côlon, de l'estomac, du poumon, du cerveau, des os, de l'oesophage, du pancréas, du sein, de l'ovaire ou de l'utérus.

14. Méthode selon l'une quelconque des revendications 11 à 13, dans laquelle ledit agent est tel que défini dans l'une quelconque des revendications 4 à 10.

15. Utilisation d'un agent capable d'inhiber la sphingosine kinase humaine dans la préparation d'un médicament pour le traitement d'un état néoplasique chez l'homme dans lequel ladite néoplasie a été transformée par surexpression de l'oncogène Ras, dans laquelle ledit agent, inhibition ou état sont tels que définis dans l'une quelconque des revendications 2 à 10.
